# EUROPEAN PATENT APPLICATION

(11) **EP 2 801 621 A1**
(43) Date of publication of application: **12.11.2014**
(21) Application number: 13166769.3
(22) Date of filing: 07.05.2013
(51) Int. Cl.: C12Q 1/68

(54) **A test kit for detecting mutations associated with limb-girdle muscular dystrophies type-2**

(71) Applicant: Latvian Biomedical Research and Study Centre, 1067 Riga (LV)
(72) Inventor: Lace, Baiba, LV-1067 Riga (LV); Jankevics, Eriks, LV-1067 Riga (LV); Inaskina, Inna, LV-1067 Riga (LV); Vasiljeva, Inta, LV-1067 Riga (LV); Stavusis, Janis, LV-1067 Riga (LV); Micule, Ieva, LV-1067 Riga (LV); Naudina, Maruta Solvita, LV-1002 Riga (LV); Strautmanis, Jurgis, LV-1004 Riga (LV)
(74) Representative: Kumaceva, Sandra

(57) **Abstract**

The present invention relates to a test for detecting Limb-Girdle Muscular Dystrophies (LGMD), especially LGMD Type-2 (LGMD-2).

A test kit for detecting mutations associated with Limb-Girdle Muscular Dystrophies Type-2 (LGMD-2), characterized in that said test kit comprises a probes selected from the group consisting of SEQ ID Nos. 1 - 78.

SEQUENCE LISTING

<110> Latvian Biomedical Research and Study Centre

<120> Probe for detection of the limb-girdle muscular dystrophies type 2

<160> 78

<170> BiSSAP 1.2

<210> 1
<211> 121
<212> DNA
<213> Homo sapiens

<220>
<221> source
<222> 1..121
<223> /mol_type="unassigned DNA"
      /organism="Homo sapiens"

<400> 1
tctgtgcagt tctggcgcta tggagagtgg gtggacgtgg ttatagatga ctgcctgcca      60

acgtacaaca atcaactggt tttcaccaag tccaaccacc gcaatgagtt ctggagtgct     120

c                                                                     121


<210> 2
<211> 120
<212> DNA
<213> Homo sapiens

<220>
<221> source
<222> 1..120
<223> /mol_type="unassigned DNA"
      /organism="Homo sapiens"

<400> 2
ctgtaatcct cccttccttc ctgcctcctc cctcctctct ccagcccatc tcttcgtttc      60

ggacagagca aacagcaaca aggagctggg tgtggaccag gagtcagagg agggcaaagg     120


<210> 3
<211> 121
<212> DNA
<213> Homo sapiens

<220>
<221> source
<222> 1..121
<223> /mol_type="unassigned DNA"
      /organism="Homo sapiens"

<400> 3
cctggcagct ctgatcagga aagtgaggaa cagcaacaat tccggaacat tttcaagcag      60

atagcaggag atgtgagtac ctccaagccc aggacgccca caggtgcttc cttctctcct     120

g                                                                     121

<210> 4
<211> 121
<212> DNA
<213> Homo sapiens

<220>
<221> source
<222> 1..121
<223> /mol_type="unassigned DNA"
      /organism="Homo sapiens"

<400> 4
aaggacctga agacacacgg gttcacactg gagtcctgcc gtagcatgat tgcgctcatg      60

gatgtatcct tcctgccgcc ccttcccgac cctctgtcat cagcccacgg gggccaaggc     120

a                                                                     121


<210> 5
<211> 121
<212> DNA
<213> Homo sapiens

<220>
<221> source
<222> 1..121
<223> /mol_type="unassigned DNA"
      /organism="Homo sapiens"

<400> 5
tccaaatcca gggggatttt gctgtgtgct gtgtagccct gacctccctc ctccagacag      60

gtggctctgg aaagctcaac ctgcaggagt tccaccacct ctggaacaag attaaggcct     120

g                                                                     121


<210> 6
<211> 121
<212> DNA
<213> Homo sapiens

<220>
<221> source
<222> 1..121
<223> /mol_type="unassigned DNA"
      /organism="Homo sapiens"

<400> 6
cagaaaattt tcaaacacta tgacacagac cagtccggca ccatcaacag ctacgagatg      60

cgaaatgcag tcaacgacgc aggtgctgag aaggaagggg tggcagggat gtggacccga     120

g                                                                     121


<210> 7
<211> 121
<212> DNA
<213> Homo sapiens

<220>
<221> source
<222> 1..121
<223> /mol_type="unassigned DNA"
      /organism="Homo sapiens"

<400> 7
cagaaggact tcttcctgta caacgcctcc aaggccagga gcaaaaccta catcaacatg     60

cgggaggtgt cccagcgctt ccgcctgcct cccagcgagt acgtcatcgt gccctccacc    120

t                                                                    121


<210> 8
<211> 121
<212> DNA
<213> Homo sapiens

<220>
<221> source
<222> 1..121
<223> /mol_type="unassigned DNA"
      /organism="Homo sapiens"

<400> 8
tggaggactt cacaggaggg gtggcagagt tttttgagat cagggatgct cctagtgaca     60

ggtacaagat catgaagaaa gccatcgaga gaggctccct catgggctgc tccattgatg    120

t                                                                    121


<210> 9
<211> 121
<212> DNA
<213> Homo sapiens

<220>
<221> source
<222> 1..121
<223> /mol_type="unassigned DNA"
      /organism="Homo sapiens"

<400> 9
gagcaggagt gctcacgatc tgtgccctgt gtctgcctgc aggggactgc tggtttctcg     60

cagccattgc ctgcctgacc ctgaaccagc accttctttt ccgagtcata ccccatgatc    120

a                                                                    121


<210> 10
<211> 121
<212> DNA
<213> Homo sapiens

<220>
<221> source
<222> 1..121
<223> /mol_type="unassigned DNA"
      /organism="Homo sapiens"

<400> 10
gagctgctgt actcctgaac catgaccctc ctctcccttc ctcctcagga catggagatc      60

tgtgcagatg agctcaagaa ggtccttaac acagtcgtga acaaacgtga gttgctcaaa     120

c                                                                     121


<210> 11
<211> 121
<212> DNA
<213> Homo sapiens

<220>
<221> source
<222> 1..121
<223> /mol_type="unassigned DNA"
      /organism="Homo sapiens"

<400> 11
gacacctcct tcctgtggtt tacctcccca tacaagacca tgaagttcat cctgtggcgg      60

cgtttccggt gggccatcat cctcttcatc atcctcttca tcctgctgct gttcctggcc     120

a                                                                     121


<210> 12
<211> 121
<212> DNA
<213> Homo sapiens

<220>
<221> source
<222> 1..121
<223> /mol_type="unassigned DNA"
      /organism="Homo sapiens"

<400> 12
atctggaata ccagagatgt gatcctggat gacctgagcc tcacggggga gaagatgagc      60

gacatttatg tgaaagggta gggagccagc gtcctcttgc ctgtccagct tcccgcagct     120

c                                                                     121


<210> 13
<211> 121
<212> DNA
<213> Homo sapiens

<220>
<221> source
<222> 1..121
<223> /mol_type="unassigned DNA"
      /organism="Homo sapiens"

<400> 13
ctccatgaca tggacgccgg tcacctgagc ttcgtggaag aggtgtttga gaaccagacc      60

cggcttcccg gaggccagtg gatctacatg agtgacaact acaccgatgt ggtaaagcag     120

g                                                                    121

<210> 14
<211> 121
<212> DNA
<213> Homo sapiens

<220>
<221> source
<222> 1..121
<223> /mol_type="unassigned DNA"
      /organism="Homo sapiens"

<400> 14
tcttcaaaag gactcttctc ccaacacgcc tctattcctt cctcagtttc cctccatgtg      60

cgaaaaaatg aggattcgta tcatagactg gtgagttctg agtcttggag tctttagggc     120

g                                                                    121

<210> 15
<211> 121
<212> DNA
<213> Homo sapiens

<220>
<221> source
<222> 1..121
<223> /mol_type="unassigned DNA"
      /organism="Homo sapiens"

<400> 15
tactccctgt ttgcggcctt ctactcagcc accatgctgc aggatgtgga tgatgccatc      60

cagtttgagg tcagcatcgg gaactacggg aacaagttcg acatgacctg cctgccgctg     120

g                                                                    121

<210> 16
<211> 121
<212> DNA
<213> Homo sapiens

<220>
<221> source
<222> 1..121
<223> /mol_type="unassigned DNA"
      /organism="Homo sapiens"

<400> 16
accaccccaa ggaaactacc ttcacgtcct ccgccccact accccgggat caaaagaaag      60

cgaagtgcgc ctacatctag aaagctgctg tcagacaaac cgcaggattt ccaggtgatg     120

a                                                                    121

<210> 17
<211> 121
<212> DNA

<213> Homo sapiens

<220>
<221> source
<222> 1..121
<223> /mol_type="unassigned DNA"
      /organism="Homo sapiens"

<400> 17
ttcccactga cgaggggcag ccagccgtcg ggggagctgc tggcctcttt tgagctcatc      60

cagagagaga aggtgaggct ggtctatatc cagatccagg aggcccaggc aggagtgggg     120

t                                                                     121


<210> 18
<211> 121
<212> DNA
<213> Homo sapiens

<220>
<221> source
<222> 1..121
<223> /mol_type="unassigned DNA"
      /organism="Homo sapiens"

<400> 18
ccgggggtga acatcaagcc tgtggtcaag gttaccgctg cagggcagac caagcggacg      60

cggatccaca agggaaacag cccactcttc aatgaggtgg gagacatggg gcatgagggc     120

c                                                                     121


<210> 19
<211> 121
<212> DNA
<213> Homo sapiens

<220>
<221> source
<222> 1..121
<223> /mol_type="unassigned DNA"
      /organism="Homo sapiens"

<400> 19
cttttcttca acttgtttga ctctcctggg gagctgtttg atgagcccat ctttatcacg      60

gtatgtctca gcagtcaaag tgttctccgt gggctgtatg tatgcacata ggtgtcagtg     120

c                                                                     121


<210> 20
<211> 121
<212> DNA
<213> Homo sapiens

<220>
<221> source
<222> 1..121
<223> /mol_type="unassigned DNA"

/organism="Homo sapiens"

<400> 20
atgatgcttt ccacccagaa tggtttgtgt ccctttttga gcagaaaaca gtgaagggct 60

ggtggccctg tgtagcagaa gagggtgaga agaaaatact ggcggtaagt ctacttcctc 120

c 121

<210> 21
<211> 121
<212> DNA
<213> Homo sapiens

<220>
<221> source
<222> 1..121
<223> /mol_type="unassigned DNA"
/organism="Homo sapiens"

<400> 21
ctactgttat tcttacctgg tcatttcctt tttgtttcac aggaaatttg cgagaatccc 60

cgatttatca ttgatggagc caacagaact gacatctgtc aaggagagct aggtaggaaa 120

g 121

<210> 22
<211> 121
<212> DNA
<213> Homo sapiens

<220>
<221> source
<222> 1..121
<223> /mol_type="unassigned DNA"
/organism="Homo sapiens"

<400> 22
cccaaattgg tcttcatcct ctctctaagg ctccatggtt cctacgaagc tctgaaaggt 60

gggaacacca cagaggccat ggaggacttc acaggagggg tggcagagtt ttttgagatc 120

a 121

<210> 23
<211> 121
<212> DNA
<213> Homo sapiens

<220>
<221> source
<222> 1..121
<223> /mol_type="unassigned DNA"
/organism="Homo sapiens"

<400> 23
accctgatga ctcggaggtg atttgcagct tcctggtggc cctgatgcag aagaaccggc 60

ggaaggaccg gaagctaggg gccagtctct tcaccattgg cttcgccatc tacgaggtgt 120

g                                                                   121

<210> 24
<211> 121
<212> DNA
<213> Homo sapiens

<220>
<221> source
<222> 1..121
<223> /mol_type="unassigned DNA"
      /organism="Homo sapiens"

<400> 24
agcgagtacg tcatcgtgcc ctccacctac gagccccacc aggaggggga attcatcctc      60

cgggtcttct ctgaaaagag gaacctctct gagtgagtgc tggcccagct ttcccacgtg     120

t                                                                   121

<210> 25
<211> 121
<212> DNA
<213> Homo sapiens

<220>
<221> source
<222> 1..121
<223> /mol_type="unassigned DNA"
      /organism="Homo sapiens"

<400> 25
gctccacgtc cacctctaac atggtcccct ccacaggatt ccacctcaac aaccagctct      60

gtgacatcat taccatgcgg tacgcagaca aacacatgaa catcgacttt gacagtttca     120

t                                                                   121

<210> 26
<211> 121
<212> DNA
<213> Homo sapiens

<220>
<221> source
<222> 1..121
<223> /mol_type="unassigned DNA"
      /organism="Homo sapiens"

<400> 26
ttctgatcta cattctgatc ttgggacttc tttcagtggc tgcagctcac catgtatgcc      60

cgaaccaggc tggcctcatc caaagccatg caggatcact caggatttca gtttcaccct     120

c                                                                   121

<210> 27
<211> 121

<212> DNA
<213> Homo sapiens

<220>
<221> source
<222> 1..121
<223> /mol_type="unassigned DNA"
      /organism="Homo sapiens"

<400> 27
gctgtgaatg cgtgcttcct ttctgggggt gcagatactt tctggaccaa ccctcagtac      60

cgtctgaagc tcctggagga ggacgatgac cctgatgact cggaggtgat ttgcagcttc     120

c                                                                     121


<210> 28
<211> 121
<212> DNA
<213> Homo sapiens

<220>
<221> source
<222> 1..121
<223> /mol_type="unassigned DNA"
      /organism="Homo sapiens"

<400> 28
agcaacttca caagaaatgt ctagaaaaga aagttcttta tgtggaccct gagttcccac      60

cggatgagac ctctctcttt tatagccaga agttccccat ccagttcgtc tggaagagac     120

c                                                                     121


<210> 29
<211> 123
<212> DNA
<213> Homo sapiens

<220>
<221> source
<222> 1..123
<223> /mol_type="unassigned DNA"
      /organism="Homo sapiens"

<400> 29
cccagagcaa ggccactgag gctgggggtg gaaacccaag tggcatctat tcagccatca      60

tcagccgcaa ttttcctatt atcggagtga aagagaagac attcgagcaa cttcacaaga     120

aat                                                                   123


<210> 30
<211> 121
<212> DNA
<213> Homo sapiens

<220>
<221> source
<222> 1..121

<223> /mol_type="unassigned DNA"
      /organism="Homo sapiens"

<400> 30
ctctggaaag ctcaacctgc aggagttcca ccacctctgg aacaagatta aggcctggca      60

ggtgggaaga gaaaatgaag cgtgggagtc aagaatgggg ttgatttgga gattcagtgt     120

g                                                                     121


<210> 31
<211> 121
<212> DNA
<213> Homo sapiens

<220>
<221> source
<222> 1..121
<223> /mol_type="unassigned DNA"
      /organism="Homo sapiens"

<400> 31
agccctcctc acctgctccc atatggctct ctctcttctt ccaacctctc aggatgtcct      60

gtgaggattt catctaccat ttcacaaagt tggagatctg caacctcacg gccgatgctc     120

t                                                                     121


<210> 32
<211> 121
<212> DNA
<213> Homo sapiens

<220>
<221> source
<222> 1..121
<223> /mol_type="unassigned DNA"
      /organism="Homo sapiens"

<400> 32
ccagcacctt cttttccgag tcatacccca tgatcaaagt ttcatcgaaa actacgcagg      60

gatcttccac ttccaggtga ggtaatgaga gtgtagttaa gagggccagc ggcaggccac     120

c                                                                     121


<210> 33
<211> 121
<212> DNA
<213> Homo sapiens

<220>
<221> source
<222> 1..121
<223> /mol_type="unassigned DNA"
      /organism="Homo sapiens"

<400> 33
agaggcggag tgcgcctgta actggcctct ggcctgtgca ttctttcaca ggagcttttc      60

ctgcatttga caaggatgga gatggtatca tcaagctcaa cgttctggag gtaaagcata 120

g 121

<210> 34
<211> 121
<212> DNA
<213> Homo sapiens

<220>
<221> source
<222> 1..121
<223> /mol_type="unassigned DNA"
/organism="Homo sapiens"

<400> 34
tttgtggaca aagatgagaa ggcccgtctg cagcaccagg tcactgagga tggagagttc 60

cggtgagtcc agaacccagg aagacccaga agggtaaggg tggggaagag aggggaaatc 120

t 121

<210> 35
<211> 121
<212> DNA
<213> Homo sapiens

<220>
<221> source
<222> 1..121
<223> /mol_type="unassigned DNA"
/organism="Homo sapiens"

<400> 35
aagtacctta ggaggcgcaa gtactccctg tttgcggcct tctactcagc caccatgctg 60

caggatgtgg atgatgccat ccagtttgag gtcagcatcg ggaactacgg gaacaagttc 120

g 121

<210> 36
<211> 121
<212> DNA
<213> Homo sapiens

<220>
<221> source
<222> 1..121
<223> /mol_type="unassigned DNA"
/organism="Homo sapiens"

<400> 36
ttggctttga agaacacaag caaaagacag acgtgcatta tcgttccctg ggaggtgaag 60

gcaacttcaa ctggaggttc attttcccct tcgactacct gccagctgag caagtctgta 120

c 121

<210> 37

<211> 121
<212> DNA
<213> Homo sapiens

<220>
<221> source
<222> 1..121
<223> /mol_type="unassigned DNA"
      /organism="Homo sapiens"

<400> 37
ctggtgtgtc accatcccca ccccgaccac caccctctgt tcagccccag aacagcctgc      60

gggacatcgt catctggatg ctgcagggag acaagcgtgt ggcataccag cgggtgcccg     120

c                                                                     121


<210> 38
<211> 125
<212> DNA
<213> Homo sapiens

<220>
<221> source
<222> 1..125
<223> /mol_type="unassigned DNA"
      /organism="Homo sapiens"

<400> 38
ttccatctgt ccgtccctca cagccagcct ctgggtgaca tccatgagac accctctgcc      60

acccacctgg accagtacct gtaccagctg cgcacccatc acctgagcca aatcactgag     120

gctgc                                                                 125


<210> 39
<211> 121
<212> DNA
<213> Homo sapiens

<220>
<221> source
<222> 1..121
<223> /mol_type="unassigned DNA"
      /organism="Homo sapiens"

<400> 39
agaaaagacc cctctgaaga caaggaggac attgaaagca acctgctccg gcccacaggc      60

gtagccctgc gaggagccca cttctgcctg aaggtcttcc gggccgagga cttgccgcag     120

a                                                                     121


<210> 40
<211> 121
<212> DNA
<213> Homo sapiens

<220>
<221> source

<222> 1..121
<223> /mol_type="unassigned DNA"
      /organism="Homo sapiens"

<400> 40
gtggatgaga aggagttcaa ccagtttgct gaggggaagc tgtctgtctt tgctgaaacc      60

gtgagtacct gccagccccc acctctgcct cccactacct ggagctgcct tggccccctg     120

c                                                                     121


<210> 41
<211> 121
<212> DNA
<213> Homo sapiens

<220>
<221> source
<222> 1..121
<223> /mol_type="unassigned DNA"
      /organism="Homo sapiens"

<400> 41
tgatttgtgt ctcctctcat tgattgcaga tggacgtggg caccatttac agagagcccc      60

gtgagttctc accactttgg ccgtatcctt gcattttggt tctggaggct gattggggac     120

a                                                                     121


<210> 42
<211> 120
<212> DNA
<213> Homo sapiens

<220>
<221> source
<222> 1..120
<223> /mol_type="unassigned DNA"
      /organism="Homo sapiens"

<400> 42
ttcctcctgc tctttcctcc ttctggcttt cagatcaggg tccaggtgat cgaggggcgc      60

cagctgccgg gggtgaacat caagcctgtg gtcaaggtta ccgctgcagg gcagaccaag     120


<210> 43
<211> 121
<212> DNA
<213> Homo sapiens

<220>
<221> source
<222> 1..121
<223> /mol_type="unassigned DNA"
      /organism="Homo sapiens"

<400> 43
caccccagag ggaggccaac atctacatgg ttcctcagaa catcaagcca gcgctccagc      60

gtaccgccat cgaggtgagc cgtccgggcc tgggcgtggg ggctgggagc agcctgccct     120

t                                                                  121

<210> 44
<211> 121
<212> DNA
<213> Homo sapiens

<220>
<221> source
<222> 1..121
<223> /mol_type="unassigned DNA"
      /organism="Homo sapiens"

<400> 44
gatctccata gggaagaaat cagtgagtga ccaggataac tacatcccct gcacgctgga      60

gcccgtattt ggaaagtaaa ttggggcatc ttgggtcttg gggtggagga gccagacagg     120

a                                                                  121

<210> 45
<211> 121
<212> DNA
<213> Homo sapiens

<220>
<221> source
<222> 1..121
<223> /mol_type="unassigned DNA"
      /organism="Homo sapiens"

<400> 45
gttttctaca tttgacttct ctctcctctc aggttcctgg gggaagccaa ggtcccactc      60

cgagaggtcc tcgccacccc tagtctgtcc gccagcttca atgcccccct gctggacacc     120

a                                                                  121

<210> 46
<211> 121
<212> DNA
<213> Homo sapiens

<220>
<221> source
<222> 1..121
<223> /mol_type="unassigned DNA"
      /organism="Homo sapiens"

<400> 46
ttccatgatc tttctctgca ggtggtagac tctcgttctc tcaggacaga tgctctcctc      60

ggggagttcc gggtaattgc ttattttcta tgaaagcagt cagttctcac ttctccgtgt     120

t                                                                  121

<210> 47
<211> 121

<212> DNA
<213> Homo sapiens

<220>
<221> source
<222> 1..121
<223> /mol_type="unassigned DNA"
      /organism="Homo sapiens"

<400> 47
ggcccggggc cttctgagcc actctcctca ttctgtgtgc ttagatcctg gcatggggcc      60

cgcggaacat gaagagttac cagctggcca acatctcctc ccccagcctc gtggtagagt     120

g                                                                     121


<210> 48
<211> 121
<212> DNA
<213> Homo sapiens

<220>
<221> source
<222> 1..121
<223> /mol_type="unassigned DNA"
      /organism="Homo sapiens"

<400> 48
tcttctccta gagggccata ggttaagatg ccttttctct ttttcttcca gggatttgaa      60

cgggacctca agggcatccc cctggaccag ggctctgagc ttcatgtggt ggtcaaagac     120

c                                                                     121


<210> 49
<211> 121
<212> DNA
<213> Homo sapiens

<220>
<221> source
<222> 1..121
<223> /mol_type="unassigned DNA"
      /organism="Homo sapiens"

<400> 49
gctgtccaca tcacctacca cgcccacctc cagggacacc cagacctgcc ccggtggctc      60

cgctacaccc agcgcagccc ccaccaccct ggcttcctct acggctctgc caccccagaa     120

g                                                                     121


<210> 50
<211> 121
<212> DNA
<213> Homo sapiens

<220>
<221> source
<222> 1..121

<223> /mol_type="unassigned DNA"
      /organism="Homo sapiens"

<400> 50
ccaggtgatg ggatcctgga gcatgacccg ttcttctgcc cacccactga ggccccagac      60

cgtgacttct tggtggatgc tctggtcacc ctcctggtgc ccctgctggt ggccctgctt     120

c                                                                     121


<210> 51
<211> 121
<212> DNA
<213> Homo sapiens

<220>
<221> source
<222> 1..121
<223> /mol_type="unassigned DNA"
      /organism="Homo sapiens"

<400> 51
cctccacttc tgtcttgcta cgacaccttg gcaccccact tccgcgttga ctggtgcaat      60

gtgaccctgg tgaggaggga ccctgggtcc gggggtgggg tggggcatgg cccccatccc     120

a                                                                     121


<210> 52
<211> 121
<212> DNA
<213> Homo sapiens

<220>
<221> source
<222> 1..121
<223> /mol_type="unassigned DNA"
      /organism="Homo sapiens"

<400> 52
aggtcacagc ctacaatcgg gacagctttg ataccactcg gcagaggctg gtgctggaga      60

ctggggaccc agaaggtacc tctagctgtg ccccatccct tccccaccaa tgccagtctt     120

g                                                                     121


<210> 53
<211> 121
<212> DNA
<213> Homo sapiens

<220>
<221> source
<222> 1..121
<223> /mol_type="unassigned DNA"
      /organism="Homo sapiens"

<400> 53
ctgggtgcag cctgaggtgt ccacctggcc ttcccaggcc ccctgctgcc ataccaagcc      60

gagttcctgg tgcgcagcca cgatgcggag gaggtgctgc cctcaacacc tgccagccgc    120

t                                                                   121

<210> 54
<211> 121
<212> DNA
<213> Homo sapiens

<220>
<221> source
<222> 1..121
<223> /mol_type="unassigned DNA"
      /organism="Homo sapiens"

<400> 54
ctgctcaacg tcacctctgc cttggaccgt gggggccgtg tcccccttcc cattgagggc    60

cgaaaagaag ggtaggtgtg caaccctaga ggacttcctg aaagaggagg atgcagcttg    120

t                                                                   121

<210> 55
<211> 121
<212> DNA
<213> Homo sapiens

<220>
<221> source
<222> 1..121
<223> /mol_type="unassigned DNA"
      /organism="Homo sapiens"

<400> 55
aaaaactgca tgccgatgtc acttgtaata gaagttttgt tgttttctac actgagcttt    60

gttgtccctt gctgaaaaac aatcttcaaa aaaaacagtt tattgtgaat atattttcaa    120

a                                                                   121

<210> 56
<211> 121
<212> DNA
<213> Homo sapiens

<220>
<221> source
<222> 1..121
<223> /mol_type="unassigned DNA"
      /organism="Homo sapiens"

<400> 56
ttttcatttc gccttcctcc tactgtgctt ttataaagag ggtggatcac tcccatgtca    60

gatacttgct taaatcgaag caggccactt tcatgaaact ccatactatc acagccattt    120

g                                                                   121

<210> 57

<211> 121
<212> DNA
<213> Homo sapiens

<220>
<221> source
<222> 1..121
<223> /mol_type="unassigned DNA"
      /organism="Homo sapiens"

<400> 57
taaatcgaag caggccactt tcatgaaact ccatactatc acagccattt ggtccaatgc      60

gaatcacggc ccaaataaca agtgttatct gaaaaagaac acaagtccac tgttggtagg     120

c                                                                     121


<210> 58
<211> 121
<212> DNA
<213> Homo sapiens

<220>
<221> source
<222> 1..121
<223> /mol_type="unassigned DNA"
      /organism="Homo sapiens"

<400> 58
tctagtgatg gaggccccaa aaggagtgga aatcaatgca gaagctggca atatggaagc      60

cacctgcagg acagagctga gactggaatc caaagatgga gaggtgaggg atgagaagga     120

c                                                                     121


<210> 59
<211> 121
<212> DNA
<213> Homo sapiens

<220>
<221> source
<222> 1..121
<223> /mol_type="unassigned DNA"
      /organism="Homo sapiens"

<400> 59
accggagcac catgcctggc tctgtggggc cacaggtata caaggtgggg atttatggct      60

ggcggaaacg atgcctgtat ttctttgtcc tgctcctcat gattttaata ctggtgaact     120

t                                                                     121


<210> 60
<211> 121
<212> DNA
<213> Homo sapiens

<220>
<221> source

<222> 1..121
<223> /mol_type="unassigned DNA"
      /organism="Homo sapiens"

<400> 60
acagaaaaag gtctaaagct agaaggagac tctgaattct tacaacctct ctacgccaaa      60

gaaatccagt cccgaccagt aagtttctgc tgagagaagg aggcattatt gttgctctag     120

a                                                                     121


<210> 61
<211> 121
<212> DNA
<213> Homo sapiens

<220>
<221> source
<222> 1..121
<223> /mol_type="unassigned DNA"
      /organism="Homo sapiens"

<400> 61
attttacttc tgctcctgat acatctttgt tttttgttta gggcctgaag gggctctttt      60

tgaacattca gtggagacac cccttgtcag agccgacccg tttcaagacc ttaggtaaga     120

a                                                                     121


<210> 62
<211> 121
<212> DNA
<213> Homo sapiens

<220>
<221> source
<222> 1..121
<223> /mol_type="unassigned DNA"
      /organism="Homo sapiens"

<400> 62
aaatctgtgt gtgtccagat gggaagctgt acctgtctgt ggccggtgtg agcaccacgt      60

gccaggagca caaccacatc tgcctctgag ctgcctgcgt cctctcggtg agctgtgcag     120

t                                                                     121


<210> 63
<211> 121
<212> DNA
<213> Homo sapiens

<220>
<221> source
<222> 1..121
<223> /mol_type="unassigned DNA"
      /organism="Homo sapiens"

<400> 63
gagtggctat ttttaatact tttttttttt ttttttgtgc ttcttttcct catctcagat      60

cagaatcccc cactcggagt ctaagcatgg atgccccaag gggtgtgcat attcaagctc     120

a                                                                     121


<210> 64
<211> 121
<212> DNA
<213> Homo sapiens

<220>
<221> source
<222> 1..121
<223> /mol_type="unassigned DNA"
      /organism="Homo sapiens"

<400> 64
tgcttaccca agctggtgca ggggacgtgg ggtccctctg gcagctcaca gagcctctac      60

gaaatctgtg tgtgtccaga tgggaagctg tacctgtctg tggccggtgt gagcaccacg     120

t                                                                     121


<210> 65
<211> 121
<212> DNA
<213> Homo sapiens

<220>
<221> source
<222> 1..121
<223> /mol_type="unassigned DNA"
      /organism="Homo sapiens"

<400> 65
ctggcggaaa aggagcggga gatggccgag atgcgggcaa ggatgcagca gcagctggac      60

gagtaccagg agcttctgga catcaagctg gccctggaca tggagatcca cgcctaccgc     120

a                                                                     121


<210> 66
<211> 121
<212> DNA
<213> Homo sapiens

<220>
<221> source
<222> 1..121
<223> /mol_type="unassigned DNA"
      /organism="Homo sapiens"

<400> 66
cacgcacgca ctagcgggcg cgtggccgtg gaggaggtgg atgaggaggg caagtttgtc      60

cggctgcgca acaagtccaa tgaggtaggc tcctgctcag ggtctaaggg gatacagctg     120

c                                                                     121

<210> 67
<211> 121
<212> DNA
<213> Homo sapiens

<220>
<221> source
<222> 1..121
<223> /mol_type="unassigned DNA"
      /organism="Homo sapiens"

<400> 67
acgaggatga ggatggagat gacctgctcc atcaccacca cggctcccac tgcagcagct      60

cgggggaccc cgctgagtac aacctgcgct cgcgcaccgt gctgtgcggg acctgcgggc     120

a                                                                     121


<210> 68
<211> 121
<212> DNA
<213> Homo sapiens

<220>
<221> source
<222> 1..121
<223> /mol_type="unassigned DNA"
      /organism="Homo sapiens"

<400> 68
ctcagctgga gctcatcaac aagcagtacc ggcggctggc ccgggagaac cgcacagaca      60

cggccagcag gctgaagcag atggtccacg agggcaacca gcgctgggac aaccttcaga     120

g                                                                     121


<210> 69
<211> 121
<212> DNA
<213> Homo sapiens

<220>
<221> source
<222> 1..121
<223> /mol_type="unassigned DNA"
      /organism="Homo sapiens"

<400> 69
gtaaaacacc ccttgcataa gtcagtgtcc aatttcacaa acttggacat aaaaatctgc      60

ccatagttgg tgaaattagg gtttaaaaca gtatgagatc agatgtcttc atatgtctct     120

g                                                                     121


<210> 70
<211> 121
<212> DNA
<213> Homo sapiens

<220>

<221> source
<222> 1..121
<223> /mol_type="unassigned DNA"
      /organism="Homo sapiens"

<400> 70
tacttgctac ccacagacaa atgcagatta tgttttttaa ggcactttat ggtgcacatg      60

cttgagaaga gatgagaaaa gcacttgctg attttcattt cagaaccatc aagaaatggg     120

g                                                                     121


<210> 71
<211> 121
<212> DNA
<213> Homo sapiens

<220>
<221> source
<222> 1..121
<223> /mol_type="unassigned DNA"
      /organism="Homo sapiens"

<400> 71
gactctcttc tgcaggtgca tgatgacgat cttttgtctt cttctgaaga ggagtgcaag      60

gataggtgcg tagtggggga gcccagggac gggctggttc tgggtccagg ctcctggccc     120

a                                                                     121


<210> 72
<211> 111
<212> DNA
<213> Homo sapiens

<220>
<221> source
<222> 1..111
<223> /mol_type="unassigned DNA"
      /organism="Homo sapiens"

<400> 72
gggtttggta aaggctccag tgtggtggcc tatgaaggac aatcctggca cgactactgc      60

ttccactgca aaaaatgctc cgtgaatctg gccaacaagc gctttgtttt c             111


<210> 73
<211> 101
<212> DNA
<213> Homo sapiens

<220>
<221> source
<222> 1..101
<223> /mol_type="unassigned DNA"
      /organism="Homo sapiens"

<400> 73
gagcgcgagg gacgcgctcg gcgggcggcg ctgctccgcg cgctgggcat gcgcctagtg      60

agctgggaag gcgggcggct ggagtggttc ggctgcaaca a 101

<210> 74
<211> 121
<212> DNA
<213> Homo sapiens

<220>
<221> source
<222> 1..121
<223> /mol_type="unassigned DNA"
/organism="Homo sapiens"

<400> 74
gtggctgcag caccagccta ggaattcccg ggcccggggg ccccgtcgtg cctctgctgc 60

cggcccccgt gtcaccgtcc tggtgcggga gttcgaggca tttgacaacg cggtgcccga 120

g 121

<210> 75
<211> 121
<212> DNA
<213> Homo sapiens

<220>
<221> source
<222> 1..121
<223> /mol_type="unassigned DNA"
/organism="Homo sapiens"

<400> 75
tacttgctac ccacagacaa atgcagatta tgttttttaa ggcactttat ggtgcacatg 60

cttgagaaga gatgagaaaa gcacttgctg attttcattt cagaaccatc aagaaatggg 120

g 121

<210> 76
<211> 121
<212> DNA
<213> Homo sapiens

<220>
<221> source
<222> 1..121
<223> /mol_type="unassigned DNA"
/organism="Homo sapiens"

<400> 76
gagctcgtct gcattgtaga agtccacacg cacagcgggc accatccagg tctggaagag 60

cgtggccagg atctgctccg caatggagtc agcaataagg tggaagtgca gagggttccg 120

t 121

<210> 77
<211> 98
<212> DNA

<213> Homo sapiens

<220>
<221> source
<222> 1..98
<223> /mol_type="unassigned DNA"
      /organism="Homo sapiens"

<400> 77
catcatgcac tttggaaaca tgaagttcaa gctgaagcag cgggaggagc aggcggaacc      60

ggacggcact gaaggtggga ggcagggatt cttggggg                              98


<210> 78
<211> 122
<212> DNA
<213> Homo sapiens

<220>
<221> source
<222> 1..122
<223> /mol_type="unassigned DNA"
      /organism="Homo sapiens"

<400> 78
aggcgaacag gaagccccag agcagggcca gtgggacgcc cagcagcgtg gacaacagac      60

tgtagcacca gtacttggag acagtgaagg tggtgtagct caccttccac acgccgtcaa     120

ag                                                                    122

## Description

### FIELD OF THE INVENTION

The present invention relates to a test kit for detecting Limb-Girdle Muscular Dystrophies (LGMD), especially LGMD Type-2 (LGMD-2).

### DESCRIPTION OF THE RELATED ART

Limb-Girdle Muscular Dystrophies (LGMD) is a heterogeneous group of muscular dystrophies that mostly affect the pelvic and shoulder girdle muscle groups. LGMD is rare with an overall incidence of 1 per 100 000 people (Yates JR, Emery AE. A population study of adult onset limb-girdle muscular dystrophy. J Med Genet 1985; 22: P250e7). LGMD is classified into two main groups, autosomal dominant (LGMD-1) and autosomal recessive (LGMD-2). The more common type is autosomal recessive with 16 genes or chromosomal loci identified to date (Rosales XQ, Tsao CY. Childhood onset of limb-girdle muscular dystrophy. Pediatr Neurol. 2012 Jan;46(1):13-23; Hicks D, et. al.). A founder mutation in Anoctamin 5 is a major cause of limb-girdle muscular dystrophy (Brain. 2011;134:171-82; Gundesli H, et. al.). Mutation in exon 1f of PLEC, leading to disruption of pectin isoform 1f, causes autosomal-recessive limb girdle muscular dystrophy (Am J Hum Genet. 2010;87:834-41). The rarer form is autosomal dominant. Ten percent of all cases of LGMD belong to this group. So far, eight genes or chromosomal loci have been associated with the autosomal dominant form (Laval SH, Bushby KM. Limb-girdle muscular dystrophies--from genetics to molecular pathology. Neuropathol Appl Neurobiol. 2004 Apr; 30(2):91-105; Greenberg SA, et. al. Etiology of limb girdle muscular dystrophy 1D/1E determined by laser capture microdissection proteomics. Ann Neurol. 2012; 71:141-5; Harms MB, et. al. Exome sequencing reveals DNAJB6 mutations in dominantly-inherited myopathy. Ann Neurol. 2012;71:407-16). Altered proteins of LGMD patients are involved in muscle cell biology in many different ways, for example CAPN3 influences IkBa/NF-kB pathway, mutations in lamin A/C cause alterations in nuclear lamina and sarcoglycan complex of muscle is built by *SCGG, SCGA, SCGB* and *SCGD* genes (Laval SH, Bushby KM. Limb-girdle muscular dystrophies-from genetics to molecular pathology. Neuropathol Appl Neurobiol. 2004 Apr; 30(2):91-105).

Diagnosis of the different types of LGMD is a challenging process due to the number of genes involved, unidentified genes at known chromosomal loci, and the lack of common mutations in non-inbred populations.

Immunostaining or immunoblotting of muscle biopsy has been historically the most used tool in establishing diagnosis for the most common types of LGMD, and in the differentiation of inflammatory myopathies. However secondary defects commonly mask primary defects of the dominant type of Limb Girdle Muscular Distrophy (LGMD1). Disadvantages of test are in low specificity and sensitivity (Tews DS, Goebel HH. Diagnostic immunohistochemistry in neuromuscular disorders. Histopathology. 2005 Jan; 46(1):1-23). The Western blot sensitivity for the most common form of LGMD, calpainopathy, is low, approximately 53%, the specificity is considerably better - 84% (Sáenz A, et. al. LGMD2A: genotype-phenotype correlations based on a large mutational survey on the calpain 3 gene. Brain. 2005 Apr; 128 (Pt 4):732-42). In addition said technique is invasive, time consuming and expensive.

Confirmation of the LGMD diagnosis as well as subtyping, which nowadays is usually done by molecular techniques. These techniques are considered to be precise, presymptomatic and non invasive. Capillary sequencing of the coding gene is used most commonly, but has certain limitations due to the size and number of all involved genes. Homozygosity mapping by genome wide SNP arrays has evolved recently for consanguineous families. This was followed by capillary sequencing to confirm diagnosis for patients with mutations in genes *TRIM32* and *FKRP* (Papić L, et. al. SNP-array based whole genome homozygosity mapping: a quick and powerful tool to achieve an accurate diagnosis in LGMD2 patients. Eur J Med Genet. 2011; Cossée M, et. al. Use of SNP array analysis to identify a novel TRIM32 mutation in limb-girdle muscular dystrophy type 2H. Neuromuscul Disord. 2009 Apr;19(4):255-60).

Exome sequencing successfully was applied for the identification of causative gene of LGMD dominant type and resulted in the new gene *DNAJB6* recognition (Harms, 2012). Joined efforts of specialists in the neuromuscular disease field worked to create NMD-chip, which aimed to develop sensitive high throughput DNA arrays for diagnosing patients affected by neuromuscular disorders, results are emerging (http://www.nmd-chip.eu). However because of the amount of genes involved (more than 30) above described techniques are expensive to check all possible mutations causing disease.

Aim of our invention was to develop a robust, low-cost mutation screening technique for the DNA diagnostics of LGMD patients.

### SUMMARY OF THE INVENTION

The present invention provides novel test kit for detecting mutations associated with Limb-Girdle Muscular Dystrophies Type-2 (LGMD-2). The test kit is characterized in that it comprises a first group of probes selected from the group consisting of SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19 and SEQ ID No. 20. The present invention also provides a test kit that additionally to the first group of probes contains a second group of probes. Said second group of probes is selected from the group consisting of SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76 and SEQ ID No. 77.

Aditionally a sequencing of *CAV3* gene (Gene ID: 859) coding region was performed. Said region has a small size of the two coding exaons for 151 amino acids. DNA samples of one hundred unrelated randomly selected persons were obtained within the framework of the national project "Genome Database of Latvian Population". Sequencing of gene *CAV3* both coding exons and exon intron boundaries was performed by using standard PCR and automatic genetic analyzer (ABI PRISM 3100, Applied Byosystems). Oligonucleotides sequences were as follows: 5'AGGGACTAACCCCACTTCC3', 5'AGGAGCGTCACAGTAGTAAG3', 5'GGCAGGCTGAAGTTATGTGG3', 5'TTTCAGGGAAACTGTGTCTGC3', 5'TCCTGCACAGATCACAGACC3 ', 5' CTTGCAGTAGCTGCCTCTTG3', 5'GTAGGGTCCAGCCACCAAG3', 5'TCATGGGGTATGGAGCAGTC 3'.

Sequencing reactions were carried out with the use of standard conditions as suggested by the manufacturer. The data acquired was processed by using programs Vector NTI packet Contig Express (Applied Byosystems).

Novel SNPs responsible for amino acid change was identified in *CAV3* gene NC_000003.11 GRCh37.p10 c.220C>T, p.R74C, and included in the list additionally to previously selected and verified twenty mutations.

Sequence, having a SEQ ID No. 78, for *CAV3* gene, mutation c.220C>T, p. R74C is as follows:

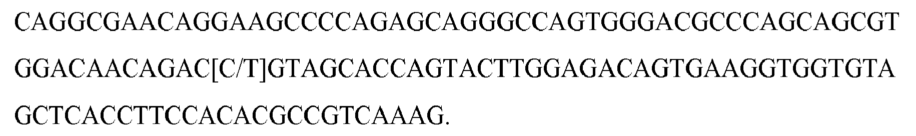

According to a novel sequence of SEQ ID No. 78 the test kit can have the first group of samples selected from the group consisting of SEQ ID Nos. 1 - 20, that further comprises a semple of SEQ ID No. 78. There can be also a test kit comprising the first group of samples selected from the group consisting of SEQ ID Nos. 1 - 20, the second group of sample selected from the group consisting of SEQ ID Nos. 21 - 77, that further comprises a sample SEQ ID No. 78.

Said sequences in general are mutations, which have diagnostic value for patients with limb girdle muscle dystrophy type 2 (LGMD-2), and are suitable for the detection.

The term "mutation" refers to DNA sequence with a certain modifications.

Mutations were selected based on their pathogenicity, the population analyzed and the allelic frequency.

Mutations, which were reported as pathogenic from functional or large control group studies, were included in the set. In the later step pathogenic mutations were cross-referenced to exclude sequence variations, which originated in patients of non-Caucasian origin. Additionally, mutations were excluded, if reported from consanguinity and inbred populations. Selected mutations later were prioritized, if reported in independent studies more than once in different countries or populations. Multiple patients from one family were considered as one case.

*CAPN3* gene 121 mutations, *DYSF* gene 50 mutations, *SGCA, SGCB, SGCD, SGCG* 28 mutations, AMELX, AMELY two gender controls, and eight polymorphism controls (altogether - 209) were initially selected based on the above mentioned criteria.

We have conducted intensive studies and found that the present invention provides a test kit for detecting Limb-Girdle Muscular Dystrophy in a most effective way.

Those and other novel features and advantages of the present invention will be now described with reference to the accompanying drawings.

### BRIEF DESCRIPTION OF THE DRAWINGS

It is to be understood that both the foregoing general description and the following detailed description are exemplary and explanatory only and are intended to provide further explanation of the present invention as claimed.
FIG. 1 shows plot of analyzed samples of p10GC against Call Rate values.
FIG. 2 shows results for one of the analyzed mutations (Seq ID No. 1) for 289 persons.
FIG. 3 shows identified *novel* missense mutation c.220C>T, p.R74C of *CAV3* gene.

### DETAILED DESCRIPTION OF THE EMBODIMENTS

In one embodiment the Illumina® VeraCode™ technique was chosen as basic technology, due to the possibility of custom designed SNP (single nucleotide polymorphisms) or mutations and deletion/duplication robust detection and broad range of multiplexing. Call rates, reproducibility, and heritability rates are above 99.9% and there is a rapid sample throughput (Veracode, 2012).

Limitations of the Illumina® VeraCode™ technique are:
1. Minimal distance between two mutations on the array >60 base pairs in both directions,
2. Detection of the triallelic and tetrallelic mutations and small inversions are not supported by the technique.

Applying above-mentioned technical limitations of the VeraCode™ platform to the selection of mutations, number of mutations for the test kit decreased to 149.

For each of the selected mutation, sequence input file was prepared according to the sequence listing design tool requirements, and final score for all of mutations was acquired. Final score described specificity and reproducibility of the PCR (Polymerase Chain Reaction) reaction for the each given mutation in numerical way.

Total number of included mutations was considerably reduced due to designs that cannot be produced with critical failure scores calculated. Failure scores are mathematical calculations by manufacturer. They are described in ranges from 0 to 1.1, with higher values reflecting greater ability to design a successful assay. Also variations with final scores below 0.4 were excluded from the list, thus increasing the overall performance of all assays, but mutation list decreased to altogether 96 sequence variations.

### Examples

The case group consisted of thirty-one patients with clinical symptoms of LGMD Type 2. Patients with predominant pelvic and shoulder girdle muscle weakness, elevated creatine kinase (CK), negative for the dystrophinopathy and electromyography (EMG) data of myopathy were included in the study. The control group consisted of 258 healthy unrelated randomly selected individuals from the Genome Database of Latvian Population. DNA was extracted by the standard phenol-chloroform method.

Genotyping for selected mutation panel was performed with customised VeraCode™ system by Illumina® on a BeadXpress™ reader also by Illumina®. Reactions were carried out under the standard conditions recommended by the manufacturer. In the given example by Illumina®.

One deviation from the standard recommendations was introduced. DNA samples in the 96 well plates were applied in the two different wells for one person, thus giving additional quality control in data analysis. For example, one 96 well plate contained DNA samples from the 48 persons.

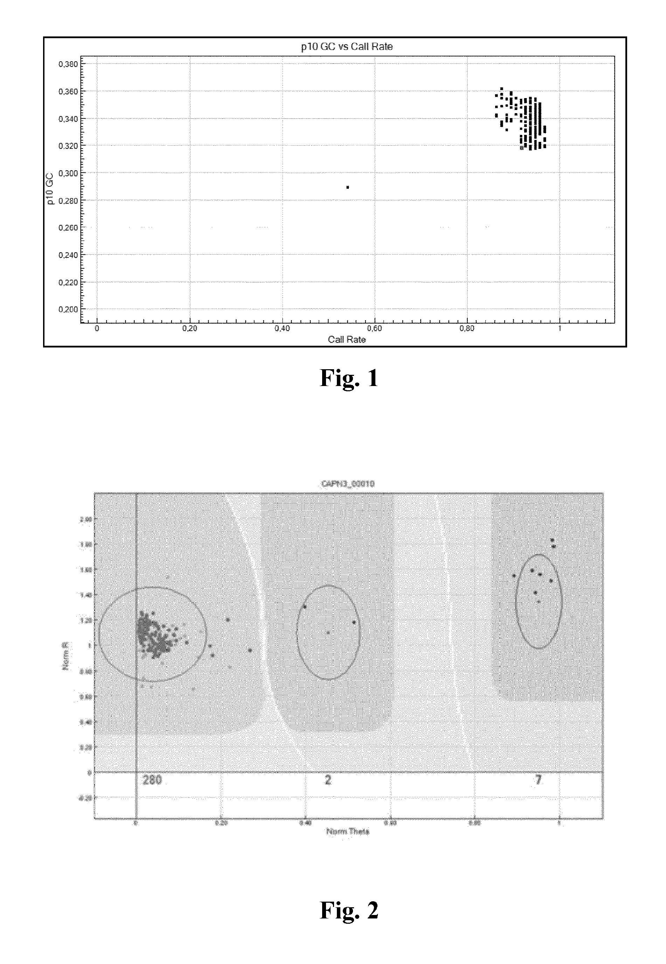

Acquired data analysis was performed by Illumina® software "Genome Studio". The first step of data quality cleaning is the assessment of manufacturer included quality markers that describe effectiveness of several important reactions. To have the best possible results, it is necessary to manually overlook each project and exclude obviously failed mutation reads. These made us exclude six of the initially chosen mutations with specific sequence listings. The next step is to make a plot of all samples rated by their Call Rate and 10% GenCall Score (p10 GC), set boundaries for each value and exclude all outlying samples. GenCall Score is a quality metric, ranging from 0-1 calculated for each data point (genotype). In this example boundaries were set as Call Rate ≥ 0.85 and p10 GC ≥ 0.320, see Figure 1. After this step 23 samples were excluded.

This was followed by mutation evaluation by following criteria Cluster Sep, Call Frequency, AB R Mean and AB T Mean, last two of which are properties of heterozygous sample cluster on Y and X axes respectively.

Additionally, "Paired Sample" test was introduced. Final cluster separation after previous adjustments must be supplemented with "Paired sample" test, which is included in Genome Studio software (http://www.illumina.com/software/genomestudio software.ilmn), but is optional in usage, and allowed combination of the both values of one sample for the final quality control. Values of remaining mutations led to exclusion of 13 mutations or sequences Nos.

96 loci were analyzed for 235 289 DNA samples. Data cleaning was completed, which decreased analyzed sample number to 212. Additionally Gen training score cut off values were set more than 0.3, that decreased number of available loci (mutations) to 77.

Results for one of the analyzed mutations (SEQ ID. No. 1 - CAPN3_00010, de1550A) for 289 persons are represented in Figure 2. There are good separation of three clusters: 280 persons are normal homozygous clustered on the right side, seven persons represent homozygous persons with identified mutation (status affected) clustered on the left side and two persons in the cluster in the middle are heterozygous mutation carriers.

Some of the markers showed good reproducibility through the all of performed experiments and manual correction for them was not necessary (n=20), but reproducibility for the rest of the mutations was not stable.

All of the final mutations were separated in the two groups using "paired sample" approach, one was a group of mutations, which constantly showed good results (SEQ ID. Nos. 1 - 20), and the second group showed fluctuating results (SEQ ID. Nos. 21 - 77). Basic metrics was obtained for the both groups separately from the Genome Studio software output. See Table 1 for the mean values of analyzed parameters. Mean values of Call Frequency, Cluster Separation and Gen Training Score were compared using unpaired t test analysis. Significantly higher mean Call Frequency was observed in the Group 1, describing better call scores above no-call threshold.

Selected twenty mutations (SEQ ID. Nos. 1 - 20) have the best call scores, they showed stable reproducibility in all the experiments, and can be used on one microarray with massive parallel multiplexing.

**Table 1. Mean values of Call Frequency, Cluster Separation and Gen Training Score and their comparison.**

| | Mean (SD) Call Frequency* | Mean (SD) Cluster Separation** | Mean (SD) Gen Training Score*** |
|---|---|---|---|
| Group 1 | 0.94 (0.22) | 0.31 (0.09) | 0.54 (0.16) |
| Group 2 | 0.7 (0.46) | 0.29 (0.09) | 0.40 (0.28) |
| P-value | 0.028 | 0.40 | 0.038 |
| 95% CI | 0.026-0.454 | 0.027-0.067 | 0.008-0.272 |

| | | | |
|---|---|---|---|
| *Call Frequency is the proportion of all samples at each locus with call scores above the no-call threshold **Cluster separation measures the separation between the three geno-type clusters in the theta dimension and varies from 0-1 ***Gen Training Score Depending on the shapes of the clusters and their relative distance to each other, a statistical score is devised | | | |

A diagnostic kit, consisting of SEQ ID. Nos. 1 - 77 with defined sequence and with proven reproducibility, is introduced. Twenty mutations set showed the best performance. Nineteen mutations did not show high specificity, or failed in repetitive samples, therefore rejected in the final step of validation.

LGMD-2 test kit is a rapid and low cost screening tool. Limb Girdle Muscle dystrophy type 2 mutation set was introduced to improve healthcare of neuromuscular disease patients. Application of this test, will allow to avoid invasive muscle biopsy, and to decrease a necessity for the hospitalization of the patients with LGMD disease.

LGMD type 2 test set is suited for the diagnostics of Limb Girdle Muscle Dystrophy, which is inherited in an autosomal recessive mode. Its main application is clinical screening test to confirm the presence of a mutation included in the test for the patient sample.

LGMD Type 2 test set can be used in all diagnostic platforms, which allow massive parallel multiplexing.

LGMD-2 test kit is particularly suited for all systems and scanners, which work with VeraCode™ systems.

A sequencing of *CAV3* gene coding region was performed. Said region has a small size of the two coding exons for 151 amino acids. DNA samples of one hundred unrelated randomly selected persons were obtained within the framework of the national project "Genome Database of Latvian Population". Sequencing of gene *CAV3* both coding exons and exon intron boundaries was performed by using standard PCR and automatic genetic analyzer (ABI PRISM 3100, Applied Byosystems). Oligonucleotides sequences were as follows: 5'AGGGACTAACCCCACTTCC3', 5'AGGAGCGTCACAGTAGTAAG3', 5'GGCAGGCTGAAGTTATGTGG3', 5'TTTCAGGGAAACTGTGTCTGC3', 5'TCCTGCACAGATCACAGACC3 ', 5' CTTGCAGTAGCTGCCTCTTG3', 5' GTAGGGTCCAGCCACCAAG3', 5'TCATGGGGTATGGAGCAGTC 3'.

Sequencing reactions were carried out with the use of standard conditions as suggested by the manufacturer. The data acquired was processed by using programs Vector NTI packet Contig Express (Applied Byosystems).

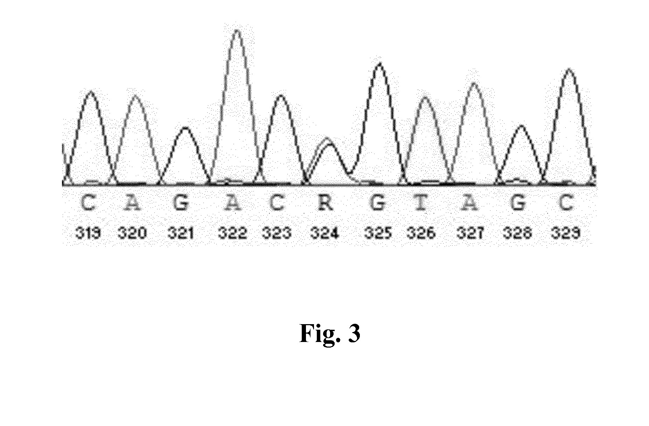

Novel SNPs responsible for amino acid change was identified c.220C>T, p.R74C, and included in the list additionally to previously selected and verified twenty mutations. In Fig. 3 is showed a identified novel missense mutation c.220C>T, p.R74C of *CAV3* gene.

Sequence, having a SEQ ID No. 78, for CAV3 gene, mutation c.220C>T, p.R74C is as follows:

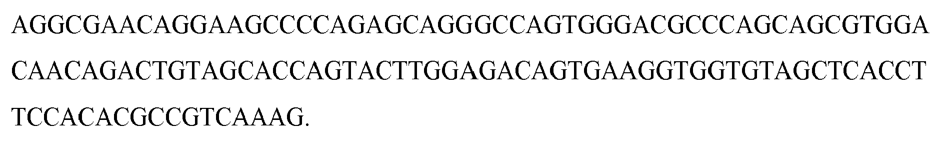

The test kits of the present invention described herein thus are useful for detecting LGMD-2 in individuals. It should be understood that the above-described embodiments and examples are merely illustrative of some of the many specific embodiments that represent the principles of the present invention. Numerous other versions can be readily devised by those skilled in the art whithout departing from the scope of the present invention.

## Claims

1. A test kit for detecting mutations associated with Limb-Girdle Muscular Dystrophies Type-2 (LGMD-2), **characterized in that** said test kit comprises a first group of probes selected from the group consisting of SEQ ID Nos. 1 - 20.

2. The test kit of claim 1, **characterized in that** said test kit further comprises a second group of probes selected from the group consisting of SEQ ID Nos. 21 - 77.

3. The kit of claim 1, **characterized in that** said kit further comprises a probe selected from SEQ ID No. 78.

4. The kit of claim 2, **characterized in that** said kit further comprises a probe selected from SEQ ID No. 78.
